# EUROPÄISCHE PATENTSCHRIFT

(11) **EP 4 043 544 B1**
(45) Veröffentlichungstag und Bekanntmachung des Hinweises auf die Patenterteilung: **03.09.2025**
(21) Anmeldenummer: 22152368.1
(22) Anmeldetag: 20.01.2022
(51) Int. Cl.: C12M 1/107, C12M 1/09, C12M 1/34, C12M 1/00

(54) **VERFAHREN UND VORRICHTUNG ZUR VERWERTUNG VON ORGANISCHEN MATERIALIEN**
METHOD AND DEVICE FOR REUSING ORGANIC MATERIAL
PROCÉDÉ ET DISPOSITIF DE VALORISATION DE MATIÈRES ORGANIQUES

(30) Priorität: 20.01.2021 DE 102021101167
(43) Veröffentlichungstag der Anmeldung: 17.08.2022
(73) Patentinhaber: WAS Wirtschaftsagentur Martin Schroeder GmbH, 50937 Köln (DE)
(72) Erfinder: LÖSCH, Walter, 98574 Schmalkalden (DE); SEEBER, Rinaldo, 07745 Jena (DE); SCHROEDER, Martin, 50677 Köln (DE)
(74) Vertreter: Lenzing Gerber Stute

(56) Entgegenhaltungen:
- EP-B1- 2 352 835
- WO-A1-2010/072220
- WO-A1-2020/044150
- DE-A1- 102005 012 367
- DE-A1- 102007 017 184
- DE-A1- 102008 015 609
- DE-A1- 102014 011 479
- DE-A1- 102018 002 883
- US-A1- 2016 186 218

## Beschreibung

Die vorliegende Erfindung betrifft ein Verfahren und ein integriertes System zur Erzeugung von Wasserstoff und Methan aus organischen Abfällen und Biomasse.

Die separate Erzeugung von Wasserstoff und Methan aus organischen Abfällen ist eine Möglichkeit, Energie zu erzeugen, ohne dass fossiler Kohlenstoff verbrannt und als CO₂ in die Atmosphäre abgegeben wird. Mikroorganismen sind in der Lage, Wasserstoff und Methan entweder durch Photosynthese oder insbesondere durch Fermentation zu produzieren. Organische Stoffe werden in der Biogaserzeugung anaerob in zwei Stufen zu Methan umgewandelt: Hydrolyse und Methanogenese. Bei der Hydrolyse entsteht Wasserstoff, der wiederum von vielen Methan erzeugenden Mikroorganismen (Methanogenen) in der zweiten Stufe des Prozesses als Elektronendonor genutzt wird. Eine Trennung der beiden Stufen ist möglich, um Wasserstoff aus der ersten Stufe zu sammeln. Die zweite Stufe dient dann zur weiteren Behandlung der verbleibenden Versauerungsprodukte. Wasserstoff und Methan können separat verwertet werden.

In der DE 10 2009 011 868 A1 ist eine Biogasanlage zur Methanisierung von Biomasse mit hohem Feststoffanteil gezeigt, die ein Faulbehältersystem (auch Fermentationsbehälter oder Fermenter genannt) mit einer Mehrzahl von gas-und flüssigkeitsdicht verschließbaren Faulbehältern umfasst. Das System umfasst einen Perkolatspeicher einen ersten und einen zweiten Perkolatbehälter. Es können auch mehr als zwei getrennte Perkolatkreisläufe vorgesehen werden. Die Anlage ist heterogen aufgebaut, das heißt die Faulbehälter sind für unterschiedliches Perkolat vorgesehen.

Die Druckschrift DE 10 2010 048 549 A1 stellt sich die Aufgabe, eine kostengünstige Leichtbau-Niedrigenergie-Biogasanlage zu schaffen. Ein Vorteil der Anlage ist es, dass bei Störungen kein kompletter Ausfall der Anlage erfolgt. Zudem kann die Anlage flexibel an anfallende Biosubstratmengen angepasst werden und weist kleinste Transporteinheiten auf.

Die Biogasanlage arbeitet nach dem Prinzip der Trockenfermentation. Bei der Anlage sind die Hydrolysestufe und die Methan produzierende Stufe nicht getrennt. Außerdem benötigt die Anlage zum Betrieb zwingend hohe Gasdrücke, sodass besonders stabile Container notwendig sind.

Die Druckschrift DE 10 2014 011 479 A1 betrifft ein Verfahren zur anaeroben Fermentation von Biomasse und dessen Umsetzung in einer Anlage mit dem Ziel der parametergesteuerten Erzeugung einer Flüssigphase. Das Verfahren ist strikt anaerob und arbeitet batchweise. Das Verfahren wird technisch in einer modularen Anlage in Containerbauweise umgesetzt. Die Einsatzstoffe werden mithilfe von speziellen Transportcontainern angeliefert und samt Container in modulare Garagen eingebracht. Es finden ausschließlich Prozesse der Hydrolyse und Acidogenese statt. Es entsteht also kein Methan, aber dennoch ein Biogas. Dieses wird in einem Containerreaktor zur hydrogenotrophen Methanogenese umgesetzt, z. B. in externen Bioreaktoren. Diese sind jedoch explizit nicht Teil der vorgeschlagenen technischen Lösung.

In der DE 10 2014 113 413 B3 ein Verfahren zum Regeln einer Biogasanlage vorgeschlagen, wobei die Biogasanlage eine Zufuhreinrichtung zur Zufuhr von einem Substrat in einen Fermenter und eine Gasverwertungseinrichtung aufweist. Der Betrieb der Biogasanlage mittels des Regelungssystems soll besonders wartungsarm sein. Eine modulare Biogasanlage ist hier nicht offenbart.

Die deutsche Patentanmeldung DE 10 2008 015 609 A1 offenbart eine Biogasanlage und ein Verfahren zur Erzeugung von Biogas, insbesondere von Methangas, in einem mehrstufigen Prozess mit einem Hydrolysevorgang und einem Methanbildungsprozess, die räumlich getrennt sind. Die Biogasanlage besitzt zumindest zwei Hydrolysebehälter und einen Gärbehälter beziehungsweise Fermenter für einen Methanbildungsprozess. Diese Biogasanlage ist nicht modular, nicht mobil und nicht besonders kompakt aufgebaut. Hinzu kommt, dass diese Biogasanlage keine Fernwartung bzw. Fernkontrolle vorsieht. Ein Umzug an einen anderen Ort bzw. ein anderer Aufbau vor Ort ist nicht vorgesehen.

Das deutsche Gebrauchsmuster DE 20 2005 012 340 U1 zeigt eine Biogasanlage mit zumindest einem Fermenter sowie Modulen, welche technische Teile der Anlage aufnehmen, insbesondere die Steuerungs- und Regelungstechnik, die Pumpentechnik sowie zumindest ein Blockheizkraftwerk. Die Biosgasanlage ist modulartig flexibel aufbaubar, da die einzelnen Module in Fertiggaragen untergebracht sind. Die Module besitzen eine gute Dichtigkeit, haben einen verbesserten Schallschutz und verminderte Schwingungsprobleme und sind kostengünstiger.

In der US 5,656,491 ist eine mobil-modulare Anlage auf einem Traktoranhänger oder Eisenbahnwaggon für die Entwicklung und Produktion von biotechnologischen Produkten im Pilotmaßstab gezeigt, wobei die Anlage aus mindestens zwei miteinander verbindbaren mobilen Modulen besteht. Die Module können in echte Produktionsmodule und in Hilfsmodule umgesetzt werden. Die Anlage erlaubt ein hohes an Flexibilität, da sie es dem Anwender ermöglicht, sich mit der Art und der Anzahl von Modulen auszustatten, die er für seine Bedürfnisse benötigt.

Die Druckschrift US 10,633,622 B2 offenbart ein Verfahren und ein integriertes System zur Vermeidung einer Kontamination von mikrobiellem Wasserstoff produzierenden Kulturen mit methanproduzierenden Kulturen bei der Wasserstoff- und Methanproduktion in einem vollständig durchmischten Bioreaktor. Verfahrenstechnisch wird ein Schwerkraftabsetzer einem Wasserstoffreaktor nachgeschaltet.

Die WO 2008/034153 A1 betrifft eine Biogasanlage mit einem Fermenter, der eine erste und zumindest eine zweite Gärkammer zum Vergären des Gärmediums sowie ein Steigrohr zwischen beiden Kammern aufweist. Des Weiteren betrifft die Erfindung ein Verfahren zum Vermischen von Gärmedium in einem Fermenter. Die Schrift beschreibt ein einstufiges Verfahren sowie eine Anlage ohne Modularität.

Stand der Technik ist es also, Wasserstoff und Methan in separaten Stufen einer Biogasanlage zu erzeugen. Der gattungsbildende Stand der Technik ist in der Druckschrift DE 10 2018 121 050 A1 offenbart und beschreibt eine modulare Anlage mit einer Anzahl von Tanks, die für die Hydrolyse oder die Fermentation eingesetzt werden können und die untereinander mittels Rohrleitungen verbunden sind, so dass zum Beispiel das Inventar von einem Hydrolysetank in einen Fermentationstank umgepumpt werden kann. Das erzeugte Biogas wird in Form von Methan in einem Gasspeicher bis zum Verbrauch gelagert.

WO 2020/044150 offenbart eine modulare Biogasanlage, die eine Vielzahl an Tanks zur Aufnahme von Biomasse umfasst. Die Vielzahl der Tanks sind fluide miteinander verbindbar.

Nachteilig beim Stand der Technik ist es, dass die Biomasse batchweise verarbeitet werden muss und das Hydrolysat aus der Wasserstoffgewinnung direkt vor Ort in eine Fermentation zur Methangewinnung überführt wird. Eventuell nicht benötigtes Methan muss dann in einem Gasspeicher gespeichert werden, bis der Verbrauch ansteht. Solche Speicher sind groß und unflexibel. Ein Transport des Methans zu anderen Verbrauchsstellen ist komplex und teuer.

Es ist deshalb Aufgabe der vorliegenden Erfindung, eine modulare, skalierbare Biogasanlage und ein Verfahren zur Biogaserzeugung zu schaffen, bei denen die Wasserstoffgewinnung und die Methangewinnung zeitlich und räumlich entkoppelt sein können. Insbesondere sollen eine Speicherung und ein Transport in Form eines Energie enthaltenden Mediums ermöglicht werden, die im Vergleich zur Methanspeicherung einfacher und flexibler erfolgen können. Diese Aufgabe wir von einer modularen Biogasanlage mit den Merkmalen des Anspruchs 1 sowie von einem Verfahren mit den Merkmalen des Anspruchs 10 gelöst.

Merkmale bevorzugter Ausführungsformen sind einzeln oder in Kombination in den abhängigen Ansprüchen aufgeführt.

Weil bei einer modularen Biogasanlage mit einer Anzahl von Tanks zur Aufnahme von Biomasse, die in einem Massestrom durch die Biogasanlage befördert wird, wobei die Tanks wenigstens zwei Hydrolysetanks umfassen, in denen die Biomasse zu einem Hydrolysat mit einem pH-Wert kleiner 7 umgesetzt wird, und die Hydrolysetanks jeweils einen Zulauf und einen Ablauf aufweisen und der Massestrom von dem Zulauf zu dem Ablauf befördert wird und in den Hydrolysetanks für eine Behandlungsdauer verweilt, vorgesehen ist, dass der Ablauf jedes Hydrolysetanks hydraulisch mit wenigstens einem Flüssigkeitsspeicher verbunden ist, in den das erzeugte Hydrolysat nach der Umsetzung eingeleitet werden kann und darin gespeichert werden kann, kann das Hydrolysat dort verweilen, bis es zur Methanerzeugung benötigt wird. So können große Energiemengen, die in Form chemischer Energie in dem Hydrolysat enthalten sind, einfach und gefahrlos gespeichert werden.

Wenn außerdem stromaufwärts des Zulaufs eine Hygienisierungsvorrichtung vorgesehen ist, in der die Biomasse vor dem Zulauf bei einer Temperatur zwischen 70°C und 120°C thermisch vorbehandelt wird, kann ein definiertes biologisches Milieu für die Hydrolyse erzeugt werden. Vorzugsweise ist dem Zulauf und/oder jeden Hydrolysetank ein Dispenser für hydrolysierende Bakterien zugeordnet, aus dem entsprechende suspendierte Bakterien in die Biomasse abgegeben werden können. Besonders vorteilhaft ist es, wenn in dem Dispenser eine Suspension zur dosierten Abgabe vorgesehen ist, die hydrolysierende Bakterien der Stämme die Clostridium acetobutyricum, Bacillus thuringiensis und/oder Clostridium butyricum enthält.

Es kann auch vorgesehen sein, dass dem Zulauf und/oder jedem Hydrolysetank ein Dispenser für pH-Regulatoren zugeordnet ist. Dadurch kann der biologische Prozess optimal eingestellt werden und/oder gezielt beeinflusst werden. Dabei ist es von Vorteil, wenn die pH-Regulatoren ein oder mehrere Reagenzien aus der Gruppe Soda, Natriumbicarbonat, Natriumhydroxid, Calciumhydroxid, Magnesiumhydroxid, Salpetersäure und Salzsäure sind. Diese Reagenzien sind in dieser Stufe der Biogasgewinnung effektiv einsetzbar und preiswert.

Bevorzugt umfasst die modulare Biogasanlage einen oder mehrere

Fermentationstanks, die stromabwärts des wenigstens einen Flüssigkeitsspeichers angeordnet sind und die hydraulisch damit verbunden sind. In diesen Fermentationstanks kann das zwischengespeicherte Hydrolysat dann gezielt zur Methangewinnung eingesetzt werden, wenn Bedarf besteht, beispielsweise zur Heizung oder zur Stromerzeugung in einem Blockheizkraftwerk oder dergleichen. Besondere Vorteile ergeben sich, wenn die Hydrolysetanks und die Fermentationstanks im Wesentlichen baugleich sind. "Im Wesentlichen" soll heißen, dass sie sich nur durch andere periphere Komponenten unterscheiden, z. B. zur Mess- und Regeltechnik, aber ansonsten baugleich sind. Es können auch jeweils ein Hydrolysetank und ein Fermentationstank in einem Standard-Transportcontainer angeordnet sind. Eine Verbindung zu einem extern angeordneten Flüssigkeitsspeicher ermöglicht dann die genannte Entkopplung von Hydrolyse und Methangewinnung.

Weil in einem Verfahren zur Erzeugung von Biogas aus Biomasse, die in einem Massestrom durch die Biogasanlage befördert wird, folgende Verfahrensschritte vorgesehen sind:
a) Sammeln von Biomasse in Form von Pflanzenresten, Lebensmittelresten oder anderen organischen Abfällen oder Rohstoffen,
b) Hydrolysieren der Biomasse während einer Verweildauer in einer Anzahl von in dem Massestrom parallel geschalteten Hydrolysetanks, wodurch die Biomasse zu einem Hydrolysat mit einem pH-Wert kleiner 7 umgesetzt wird,
c) Auffangen des im Schritt b) gebildeten Gases und Trennen des entstehenden Wasserstoffs von anderen Gasen, insbesondere von CO₂,
d) Überführen von im Schritt c) gebildetem flüssigem Hydrolysats aus jedem der Hydrolysetanks in einen gemeinsamen, stromabwärts der Hydrolysetanks liegenden Flüssigkeitsspeicher,
e) Abgabe des Hydrolysats an einen oder mehrere Fermenter zur Gewinnung von Methan aus dem Hydrolysat, insbesondere unter Zugabe von Methan erzeugenden Bakterienkulturen,
können ebenfalls die oben im Zusammenhang mit der vorgeschlagenen modularen Biogasanlage beschriebenen Vorteile erzielt werden.

Auch hier ist es von Vorteil, wenn vor dem Schritt a) die Biomasse in einer Hygienisierungsvorrichtung bei einer Temperatur zwischen 70°C und 120°C thermisch vorbehandelt wird. Weiter ist es von Vorteil für einen Betrieb unter kontrollierten biologischen Bedingungen, wenn der Biomasse zu Beginn des Schritts b) hydrolysierende Bakterien zugegeben werden, insbesondere Clostridium acetobutyricum, Bacillus thuringiensis und/oder Clostridium butyricum.

Die biologische Aktivität wird gestoppt, wenn die Biomasse zum Abschluss der Hydrolyse und vor der Abgabe an den Flüssigkeitsspeicher hygienisiert wird. Dies kann durch Temperatureinwirkung geschehen, aber auch dadurch, dass die genannten Bakterien sämtliche Nährstoffe verbraucht haben und dann absterben oder inaktiv werden.

Vorteilhaft kann auch das Hydrolysat im Schritt e) mittels Tanktransport an eine von dem Hydrolysator entfernt angeordneten Methan erzeugende Einrichtung transportiert werden. Das Hydrolysat, das beispielsweise Carbonsäuren und Alkohole in flüssiger Form enthält, kann so als Energieträger verkauft werden.

Der im Schritt c) gewonnene Wasserstoff aus der Hydrolyse kann mit Vorteil am Ort der Verfahrensdurchführung beispielsweise mittels einer Brennstoffzelle zur Stromerzeugung verwendet werden und so der erzeugte Strom zum Betrieb der Anlage eingesetzt werden.

Ein Vorteil kann sich auch ergeben, wenn die Biomasse mit einem Kraftfahrzeug zur Hydrolyse transportiert wird und die vor dem Schritt a) vorgesehene Hygienisierung unter Verwendung der Abwärme eines Verbrennungsmotors des Kraftfahrzeugs zumindest teilweise während des Transports erfolgt. Dann wird der für die Hygienisierung notwendige Energieeinsatz minimiert. Die Hygienisierung ist zum Beispiel bei der Sammlung von Lebensmittelabfällen aus der Gastronomie oder der Lebensmittelherstellung, aber auch bei der Sammlung von Tierexkrementen wichtig.

Nachfolgend werden zwei bevorzugte Ausführungsformen der Erfindung unter Bezugnahme auf die Zeichnung beschrieben. Es zeigen:
FIG. 1 ist ein Blockdiagramm, das einen Verfahrensablauf unter Nutzung einer Vorrichtung nach der vorliegenden Erfindung zeigt; sowie
FIG. 2 zeigt einen anderen Verfahrensablauf, der sich von dem in Fig. 1 gezeigten dadurch unterscheidet, dass andere Biomasse und eine andere Methanverwertung vorgesehen sind.

Im Allgemeinen sind die hier beschriebenen Ausführungsbeispiele auf ein integriertes System zur Wasserstoff- und Methanproduktion aus organischen Abfällen und Biomasse gerichtet. Wenn von einem Massestrom gesprochen wird, so ist hier nicht ein kontinuierlicher Strom gemeint, sondern vielmehr eine batchweise diskontinuierliche Betriebsart, bei der die Biomasse jedoch von einer Station zu der nächsten befördert, gepumpt oder sonst wie transportiert wird.

Der Begriff "vollständig gemischter Bioreaktor" bezeichnet einen mechanisch oder hydraulisch durchmischten Behälter, der Mikroorganismen in Suspension und ein Wachstumsmedium enthält, das typischerweise aus Nährstoffen wie organischem Kohlenstoff, stickstoffhaltigen Verbindungen, phosphorhaltigen Verbindungen und mineralischen Spurenlösungen besteht.

Der Begriff "Fermentationstank" bezieht sich auf alle gängigen Konstruktionen, die für die anaerobe Umwandlung von organischen Abfällen in Methan und Kohlendioxid verwendet werden. Zu den Fermentationstanks gehören u. a. einstufige kontinuierlich gerührte Tankreaktoren.

Der Begriff "wasserstoffproduzierende Mikroorganismen" bezeichnet Mikroorganismen, die in der Lage sind, organische Stoffe unter anaeroben Bedingungen zu fermentieren, um Wasserstoff, Kohlendioxid und eine Vielzahl organischer Säuren und Alkohole zu erzeugen.

Der Ausdruck "organischer Abfall" bezieht sich auf Abfälle, die Kohlenstoff und Wasserstoff enthalten, wie z. B. Alkohole, Ketone, Aldehyde, flüchtige Fettsäuren, Ester, Carbonsäuren, Ether, Kohlenhydrate, Proteine, Lipide, Polysaccharide, Monosaccharide, Zellulose und Nukleinsäuren, aber nicht darauf beschränkt.

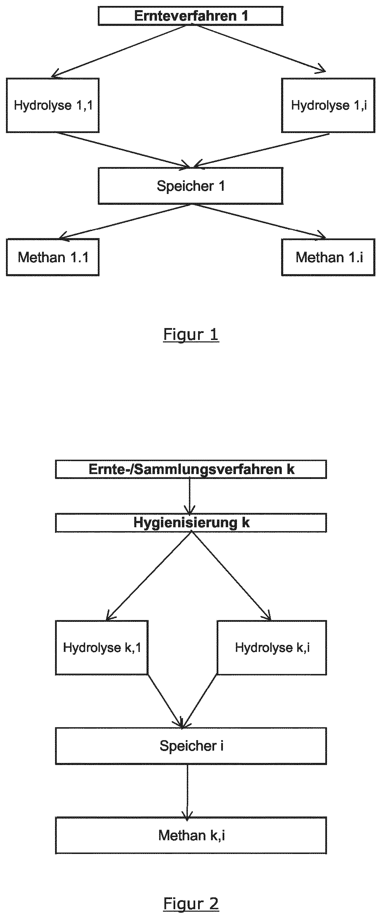

In Fig. 1 ist schematisch ein Verfahrensablauf dargestellt, bei dem in einem Ernteverfahren 1 zunächst Biomasse gewonnen wird, die sich biologisch in einem Zustand befindet, der eine direkte Hydrolyse erlaubt. Die Biomasse ist also nicht mit einem bakteriellen Milieu belastet, das die biologischen Vorgänge in einer Hydrolyse beeinträchtigen würde. Beispiele hierfür sind Grünabfälle, wie sie an kommunalen Grünabfallsammelstellen anfallen, Rasenschnitt, oder auch landwirtschaftliche Erzeugnisse, die nicht zum menschlichen oder tierischen Verzehr geeignet sind. Diese Stoffe werden also in dem Ernteverfahren 1 gesammelt und, falls erforderlich, mechanisch aufbereitet, zerkleinert, gesäubert und in ihrem Wassergehalt eingestellt. Sodann werden diese Stoffe als Biomasse in eine Anzahl i von Hydrolysetanks 1,1 bis 1,i gegeben. Vorzugsweise ist die Biomasse zuvor so eingestellt worden, dass sie pumpfähig ist. Sie kann dann aus einem Vorratsbehälter oder einem Sammelbehälter in die Hydrolysetanks umgepumpt werden.

Diese Hydrolysetanks selbst sind als Module baugleich ausgeführt. Sie umfassen einen Zulauf und einen Ablauf für die Biomasse und das nach der Hydrolyse vorliegende Inventar, sowie einen Gasanschluss, durch den das bei der Hydrolyse anfallende Gas abgeleitet und gesammelt werden kann. In diesem Gas sind im wesentlichen Wasserstoff und Kohlendioxid enthalten. Die Hydrolysetanks sind vorzugsweise als Kunststoffbehälter ausgeführt und weisen ein Volumen etwa im Bereich von 1 m³ bis 3 m³ auf. Auch größere Hydrolysetanks sind denkbar. Die geometrische Form der Hydrolysetanks ist so zu wählen, dass sie in eine Hälfte eines kommerziellen 20 Fuß-Transportcontainer passt.

Jedem Hydrolysetank sind Sensoren und/oder Aktoren für die Messe und Regeltechnik des Prozesses zugeordnet. Diese Sensoren können Temperatursensoren, pH-Wert-Sensoren, Füllstandssensoren und Drucksensoren umfassen. Die Aktoren sind vorzugsweise eine Vorrichtung zur Durchmischung des Inventars, insbesondere ein Rührwerk, und gegebenenfalls eine Heizvorrichtung zur Einstellung einer gewünschten Temperatur des Inventars, insbesondere zu Beginn des biologischen Prozesses.

Außerdem sind jedem Hydrolysetank Dosiervorrichtungen zugeordnet, mit denen gezielt Suspensionen von bestimmten Bakterienstämmen, die für die Hydrolyse besonders geeignet sind, zudosiert werden können. Außerdem ist es vorteilhaft, wenn in einer separaten Dosiervorrichtung Stoffe zur Regulierung des pH-Werts in dem Hydrolysetank enthalten sind, die gegebenenfalls dem Inventar zugegeben werden können. Die geeigneten Bakterienstämme und pH-Wert-Regulatoren sind in der Beschreibungseinleitung genannt.

Mit diesen Randbedingungen kann das in die Hydrolysetanks 1,1 bis 1,i überführte Inventar biologisch umgesetzt werden. Dabei werden insbesondere langkettige Kohlenwasserstoffe (Fette) aufgespalten, sodass sich Carbonsäuren wie Essigsäure und Propionsäure sowie Alkohole bilden. Bei dieser Aufspaltung entsteht Kohlendioxid und Wasserstoff. Diese beiden Gase können relativ einfach aufgrund der unterschiedlichen Dichte getrennt werden, sodass der Wasserstoff aufgefangen und beispielsweise als Energieversorgung zum Betrieb der Biogasanlage eingesetzt werden kann.

Wenn der biologische Prozess abgeschlossen ist, also die Umsetzung in Carbonsäuren und Alkohole nahezu vollständig durchgeführt und/oder die Gasproduktion zum Erliegen gekommen ist, wird das so verarbeitete Inventar in einen Speicher 1 überführt. Dort liegt die Biomasse als saure Flüssigkeit vor, die einen erheblichen Anteil an Kohlenwasserstoffen enthält und somit auch einen relativ hohen chemischen Energieinhalt aufweist. Die Flüssigkeit ist ohne weiteres langfristig lagerfähig und muss nicht sofort verarbeitet werden. Sie kann auch aufgrund ihres Energiegehalts gehandelt, verkauft und transportiert werden, falls eine Verwertung vor Ort nicht erforderlich oder nicht möglich ist.

Bei dem Verfahren gemäß Figur 1 ist jedoch eine Weiterverarbeitung vor Ort vorgesehen. Dazu kann, sobald ein Bedarf an Methan besteht, der Inhalt des Speichers 1 in Fermentationstanks "Methan 1.1 bis 1.i" überführt werden. In diesen Fermentationstanks wird nun die aus dem Speicher kommende Biomasse biologisch fermentieren, sodass die enthaltenen Kohlenwasserstoffe weitgehend in Wasser, gelöste Mineralien, Methan und Kohlendioxid umgesetzt werden. Dieser Prozess ist aus der Biogasproduktion bekannt und muss hier nicht näher erläutert werden. Auch die gegebenenfalls zuzugebenden Bakterienstämme und eine eventuell vorteilhafte Temperatursteuerung sind bekannt.

Bei den bekannten Verfahren wird jedoch die Methanerzeugung so lange fortgeführt, bis die Methanbildung im Wesentlichen zum Erliegen gekommen ist. Die dabei gebildeten flüssigen Reste der Biomasse sind nahezu anorganisch und weisen einen hohen Gehalt an Nitraten auf. Diese Reststoffe werden in der Landwirtschaft gerne zur Düngung von Äckern verwendet. Diese Praxis ist in mehrerer Hinsicht problematisch. Zum einen wird häufig nicht nach dem Bedarf der angebauten Pflanzen gedüngt, sondern der Reststoff im Überschuss ausgebracht, sodass unverbrauchte Reste nicht im Boden gebunden werden können, sondern vielmehr in das Grundwasser oder in Oberflächengewässer gelangen. Die Wasserqualität wird dadurch signifikant beeinträchtigt. Zum anderen haben diese nitrathaltigen, anorganischen Reste einen hohen Sauerstoffgehalt, der bei Ausbringung den pH-Wert des Bodens negativ beeinflusst.

In dem vorliegend beschriebenen Verfahren wird deshalb vorzugsweise die Fermentation in den einzelnen Fermentationstanks hinsichtlich ihrer Methanproduktion überwacht und bei Erreichen der maximalen Methanproduktion pro Zeiteinheit oder kurz danach abgebrochen. Der sich dann in den Fermentationstanks befindende Reststoff enthält noch einen nennenswerten organischen Anteil, insbesondere Fasern und Feststoffe. Dieser Reststoff kann aus dem anaeroben Zustand in einen aeroben Zustand überführt werden, in dem er belüftet wird, und dann in eine aerobe Rotte gebracht werden. In dieser aeroben Rotte werden die anaeroben und potenziell pathogenen Bakterien aus der Fermentation inaktiviert. Aerobe Bakterien und Pilze zersetzen die organischen Reststoffe weiter und bilden dabei ein hygienisiertes, trockenes und mit einem hohen Faseranteil versehenes Substrat. Dieses Substrat kann dann sehr vorteilhaft zur Bodenverbesserung eingesetzt werden, beispielsweise in Gegenden, in denen die Böden durch einen hohen Sandgehalt gekennzeichnet sind und nur eine geringe Wasseraufnahmefähigkeit besitzen. Das Substrat kann, wenn es in diese Böden eingearbeitet wird, die Wasseraufnahmefähigkeit verbessern und diese Böden dadurch landwirtschaftlich mit höheren Erträgen besser nutzbar machen. Ein wirtschaftlicher Aspekt besteht darin, dass das so hergestellte Substrat auch in kleineren Mengen lokal vermarktet werden kann, denn es kann beispielsweise anstelle des aus Umweltaspekten äußerst bedenklichen, aus trockengelegten Moorgebieten stammenden Torfs auch im Gartenbereich eingesetzt werden.

Das in den Fermentationstanks "Methan 1.1" - "Methan 1.i" gewonnene Methan wird aufgefangen und beispielsweise in einem Blockheizkraftwerk zur Erzeugung von Strom und Wärme verwertet. Es kann auch komprimiert und zur Betankung von Fahrzeugen genutzt werden, deren Verbrennungsmotor für den Betrieb mit Erdgas eingerichtet ist.

Die Fermentationstanks sind vorzugsweise im Wesentlichen baugleich mit den Hydrolysetanks, die weiter oben erwähnt wurden. Insbesondere kann die Biogasanlage so aufgebaut sein, dass jeweils ein Hydrolysetank und ein Fermentationstank in einem Standard-Transportcontainer oder einem Rahmen mit den ungefähren Abmessungen eines solchen Containers angeordnet werden können. Dieser Transportcontainer enthält dann auch die unmittelbar zum Betrieb der beiden Tanks erforderlichen Steuer-und Regelvorrichtungen, sodass jeder Transportcontainer an sich eine eigenständige Biogasanlage darstellt. Die Modularität ergibt sich dadurch, dass mehrere Transportcontainer 1-i parallel vorgesehen sein können und den eingehenden Biomassestrom batchweise parallel verarbeiten können. Dieser Aufbau hat den Vorteil, dass hinsichtlich der Ausfallsicherheit des einzelnen Moduls kein übermäßig hoher Aufwand betrieben werden muss, da der gelegentliche Ausfall eines Moduls nicht zum Ausfall der gesamten Biogasanlage führt. Dementsprechend können auch für den Bereich der Steuerung und Regelung der Module einfachere Komponenten gewählt werden, da deren Ausfallsicherheit nicht den Ansprüchen genügen muss, die bei einer großen, kommerziellen, nicht modularen Biogasanlage erforderlich sind.

Wenn ein Hydrolysetank und ein Fermentationstank in demselben Container angeordnet sind, kann zwischen diesen beiden Elementen ein Abzapf vorgesehen sein, um das Inventar nach der Hydrolyse und vor der Fermentation in den Speicher 1 zu überführen. Dort kann das Inventar dann verweilen, bis es zur Methanproduktion benötigt wird. In diesem Fall kann dann das Inventar aus dem externen Speicher 1 in die Fermentationstanks 1 bis i überführt werden. Dieser Vorgang kann einfach durch Pumpen durchgeführt werden.

Die Figur 2 zeigt einen anderen Verfahrensablauf, bei dem zunächst das Ernte-/Sammlungsverfahren k mit anderen biologischen Materialien durchgeführt wird. Hier wird zum Beispiel Abfall aus der Lebensmittelproduktion, aus der Tierhaltung und/oder aus der Gastronomie gesammelt. Dieser Abfall ist möglicherweise biologisch nicht in einem Zustand, der eine unmittelbare Hydrolyse erlauben würde, da die Stoffe ein bakterielles Milieu aufweisen, die die Hydrolyse stören können. Deshalb ist im Verfahrensablauf stromabwärts nach dem Ernte-und Sammlungsverfahren k eine Hygienisierung k vorgesehen, bei der die in der gesammelten Biomasse enthaltenen Mikroorganismen weitgehend inaktiviert oder abgetötet werden. Diese Hygienisierung kann zum Beispiel bereits während des Transports von der Sammelstelle zur Biogasanlage erfolgen. Dies ist weiter oben in der Beschreibungseinleitung im Detail näher beschrieben. Die Hygienisierung erfolgt vorzugsweise thermisch bei Temperaturen zwischen 70 °C und 120 °C.

Mit den hygienisierten Ausgangsstoffen wird danach im Prinzip weiter verfahren, wie es oben zu Figur 1 beschrieben wurde. Die Biomasse wird also mechanisch vorbereitet und hinsichtlich des Wassergehalts eingestellt und dann in die verschiedenen Hydrolysetanks überführt. Dort wird die Hydrolyse durchgeführt und das anfallende Gas gesammelt und verwertet. Die aus der Hydrolyse stammende Flüssigkeit, die auch hier neben anderen Inhaltsstoffen hauptsächlich Carbonsäuren und Alkohole enthält, wird gespeichert, bis eine Verwertung durch Fermentation erfolgen kann. Hier ist in diesem Ausführungsbeispiel vorgesehen, dass die Methanerzeugung alternativ zu der in Figur 1 beschriebenen Verwertung in einzelnen Fermentationstanks auch in einer anderen modularen oder nicht modularen Biogasanlage durchgeführt werden kann. Dazu kann wie oben beschrieben das Hydrolysat in flüssiger Form als Energieträger gehandelt und transportiert werden oder vor Ort durch Fermentation verwertet werden.

Die Vorrichtung umfasst vorzugsweise auch Temperaturregler zur Steuerung der Temperatur, wobei ein typischer Temperaturbereich, in dem die Temperatur des Inhalts sowohl der Hydrolyse als auch der Fermentation gehalten wird, liegt zwischen etwa 20° C. und etwa 60° C.

Zusammenfassend lässt sich sagen, dass die hier offenbarte Methode und Vorrichtung zur Wasserstoff- und Methanproduktion aus organischen Abfällen und Biomasse aus mehreren Gründen sehr vorteilhaft ist.

Der Ablauf der Hydrolysetanks ist mit kurzkettigen Carbonsäuren beladen, die durch den mikrobiologischen Abbau der einfließenden Abfallbestandteile durch wasserstoffproduzierende Bakterien entstanden sind, und ist ein hervorragendes Substrat für methanbildende Bakterien. Dieses Substrat kann einerseits gespeichert und ggf. transportiert werden, und es ermöglicht andererseits eine besonders hohe Effizienz der nachfolgenden Fermentation und Methangewinnung, indem es die volumetrische Methanproduktionsrate, die Methanausbeute und die Effizienz der Feststoffzerstörung erhöht und somit höhere volumetrische und organische Beladungsraten ermöglicht.

## Patentansprüche

1. Modulare Biogasanlage mit einer Anzahl von Tanks zur Aufnahme von Biomasse, die in einem Massestrom durch die Biogasanlage befördert wird, wobei die Tanks wenigstens zwei Hydrolysetanks umfassen, in denen die Biomasse zu einem Hydrolysat mit einem pH-Wert kleiner 7 umgesetzt wird, und die Hydrolysetanks jeweils einen Zulauf und einen Ablauf aufweisen und der Massestrom von dem Zulauf zu dem Ablauf befördert wird und in den Hydrolysetanks für eine Behandlungsdauer verweilt, **dadurch gekennzeichnet, dass** der Ablauf jedes Hydrolysetanks hydraulisch mit wenigstens einem Flüssigkeitsspeicher verbunden ist, in den das erzeugte Hydrolysat nach der Umsetzung eingeleitet werden kann und darin gespeichert werden kann.

2. Modulare Biogasanlage nach Anspruch 1, **dadurch gekennzeichnet, dass** stromaufwärts des Zulaufs eine Hygienisierungsvorrichtung vorgesehen ist, in der die Biomasse vor dem Zulauf bei einer Temperatur zwischen 70°C und 120°C thermisch vorbehandelt wird.

3. Modulare Biogasanlage nach Anspruch 1 oder 2, **dadurch gekennzeichnet, dass** dem Zulauf und/oder jeden Hydrolysetank ein Dispenser für hydrolysierende Bakterien zugeordnet ist.

4. Modulare Biogasanlage nach Anspruch 3, **dadurch gekennzeichnet, dass** in dem Dispenser eine Suspension zur dosierten Abgabe vorgesehen ist, die hydrolysierende Bakterien der Stämme die Clostridium acetobutyricum, Bacillus thuringiensis und/oder Clostridium butyricum enthält.

5. Modulare Biogasanlage nach einem der vorhergehenden Ansprüche, **dadurch gekennzeichnet, dass** dem Zulauf und/oder jeden Hydrolysetank ein Dispenser für pH-Regulatoren zugeordnet ist.

6. Modulare Biogasanlage nach Anspruch 5, **dadurch gekennzeichnet, dass** die pH-Regulatoren ein oder mehrere Reagenzien aus der Gruppe Soda, Natriumbicarbonat, Natriumhydroxid, Calciumhydroxid, Magnesiumhydroxid, Salpetersäure und Salzsäure sind.

7. Modulare Biogasanlage nach einem der vorhergehenden Ansprüche, **dadurch gekennzeichnet, dass** die Tanks einen oder mehrere Fermentationstanks umfassen, die stromabwärts des wenigstens einen Flüssigkeitsspeichers angeordnet sind und die hydraulisch damit verbunden sind.

8. Modulare Biogasanlage nach Anspruch 7, **dadurch gekennzeichnet, dass** die Hydrolysetanks und die Fermentationstanks im Wesentlichen baugleich sind.

9. Modulare Biogasanlage nach Anspruch 8, **dadurch gekennzeichnet, dass** jeweils ein Hydrolysetank und ein Fermentationstank in einem Standard-Transportcontainer angeordnet sind.

10. Verfahren zur Erzeugung von Biogas aus Biomasse, die in einem Massestrom durch die Biogasanlage befördert wird, **gekennzeichnet durch** folgende Verfahrensschritte:
a) Sammeln von Biomasse in Form von Pflanzenresten, Lebensmittelresten oder anderen organischen Abfällen oder Rohstoffen,
b) Hydrolysieren der Biomasse während einer Verweildauer in einer Anzahl von in dem Massestrom parallel geschalteten Hydrolysetanks, wodurch die Biomasse zu einem Hydrolysat mit einem pH-Wert kleiner 7 umgesetzt wird,
c) Auffangen des im Schritt b) gebildeten Gases und Trennen des entstehenden Wasserstoffs von anderen Gasen, insbesondere von CO₂,
d) Überführen von im Schritt c) gebildetem flüssigem Hydrolysats aus jedem der Hydrolysetanks in einen gemeinsamen, stromabwärts der Hydrolysetanks liegenden Flüssigkeitsspeicher,
e) Abgabe des Hydrolysats an einen oder mehrere Fermenter zur Gewinnung von Methan aus dem Hydrolysat, insbesondere unter Zugabe von Methan erzeugenden Bakterienkulturen.

11. Verfahren nach Anspruch 10, **dadurch gekennzeichnet, dass** vor dem Schritt a) die Biomasse in einer Hygienisierungsvorrichtung bei einer Temperatur zwischen 70°C und 120°C thermisch vorbehandelt wird.

12. Verfahren nach Anspruch 10 oder 11, **dadurch gekennzeichnet, dass** der Biomasse zu Beginn des Schritts b) hydrolysierende Bakterien zugegeben werden, insbesondere Clostridium acetobutyricum, Bacillus thuringiensis und/oder Clostridium butyricum.

13. Verfahren nach einem der vorhergehenden Ansprüche 10 bis 12, **dadurch gekennzeichnet, dass** die Biomasse zum Abschluss der Hydrolyse und vor der Abgabe an den Flüssigkeitsspeicher hygienisiert wird.

14. Verfahren nach einem der vorhergehenden Ansprüche 10 bis 13, **dadurch gekennzeichnet, dass** das Hydrolysat im Schritt e) mittels Tanktransport an eine von dem Hydrolysator entfernt angeordneten Methan erzeugende Einrichtung transportiert wird.

15. Verfahren nach einem der vorhergehenden Ansprüche 10 bis 14, **dadurch gekennzeichnet, dass** der im Schritt c) gewonnene Wasserstoff am Ort der Verfahrensdurchführung beispielsweise mittels einer Brennstoffzelle zur Stromerzeugung verwendet wird und dass der erzeugte Strom zum Betrieb der Anlage eingesetzt wird.

16. Verfahren nach einem der vorhergehenden Ansprüche 11 bis 15, **dadurch gekennzeichnet, dass** die Biomasse mit einem Kraftfahrzeug zur Hydrolyse transportiert wird und dass die vor dem Schritt a) vorgesehene Hygienisierung unter Verwendung der Abwärme eines Verbrennungsmotors des Kraftfahrzeugs zumindest teilweise während des Transports erfolgt.

## Claims

1. Modular biogas plant having a number of tanks for receiving biomass which is conveyed in a mass flow through the biogas plant, wherein the tanks comprise at least two hydrolysis tanks, in which the biomass is converted to a hydrolysate having a pH value of less than 7, and the hydrolysis tanks each have an inlet and an outlet and the mass flow is conveyed from the inlet to the outlet and remains in the hydrolysis tanks for a treatment period, **characterized in that** the outlet of each hydrolysis tank is hydraulically connected to at least one liquid storage tank into which the hydrolysate produced can be introduced after the conversion and can be stored therein.

2. Modular biogas plant according to claim 1, **characterized in that** a hygienization device is provided upstream of the inlet, in which the biomass is thermally pretreated at a temperature between 70°C and 120°C upstream the inlet.

3. Modular biogas plant according to claim 1 or 2, **characterized in that** a dispenser for hydrolysing bacteria is assigned to the inlet and/or each hydrolysis tank.

4. Modular biogas plant according to claim 3, **characterized in that** a suspension for metered delivery is provided in the dispenser, the suspension containing hydrolysing bacteria of the strains Clostridium acetobutyricum, Bacillus thuringiensis and/or Clostridium butyricum.

5. Modular biogas plant according to one of the preceding claims, **characterized in that** a dispenser for pH regulators is assigned to the inlet and/or each hydrolysis tank.

6. Modular biogas plant according to claim 5, **characterized in that** the pH regulators are one or more reagents from the group soda, sodium bicarbonate, sodium hydroxide, calcium hydroxide, magnesium hydroxide, nitric acid and hydrochloric acid.

7. Modular biogas plant according to one of the preceding claims, **characterized in that** the tanks comprise one or more fermentation tanks which are arranged downstream of the at least one liquid storage tank and which are hydraulically connected thereto.

8. Modular biogas plant according to claim 7, **characterized in that** the hydrolysis tanks and the fermentation tanks are essentially identical in construction.

9. Modular biogas plant according to claim 8, **characterized in that** a hydrolysis tank and a fermentation tank are each arranged in a standard transport container.

10. Method for the production of biogas from biomass which is conveyed in a mass flow through the biogas plant, **characterized by** the following method steps:
a) Collecting biomass in the form of plant residues, food waste or other organic waste or raw materials,
b) Hydrolyzing the biomass during a residence time in a number of hydrolysis tanks connected in parallel in the mass flow, whereby the biomass is converted to a hydrolysate with a pH value of less than 7,
c) Collecting the gas formed in step b) and separating the resulting hydrogen from other gases, in particular CO₂,
d) Transferring liquid hydrolysate formed in step c) from each of the hydrolysis tanks to a common liquid storage tank located downstream of the hydrolysis tanks,
e) Delivery of the hydrolysate to one or more fermenters for the recovery of methane from the hydrolysate, in particular with the addition of methane-producing bacterial cultures.

11. Method according to claim 10, **characterized in that**, prior to step a), the biomass is thermally pretreated in a hygienizing device at a temperature of between 70°C and 120°C.

12. Method according to claim 10 or 11, **characterized in that** hydrolysing bacteria, in particular Clostridium acetobutyricum, Bacillus thuringiensis and/or Clostridium butyricum, are added to the biomass at the beginning of step b).

13. Method according to any of the preceding claims 10 to 12, **characterized in that** the biomass is hygienized at the end of the hydrolysis and before being delivered to the liquid storage.

14. Method according to any one of the preceding claims 10 to 13, **characterized in that** the hydrolysate is transported in step e) by means of tank transport to a methane-producing device arranged remotely from the hydrolyzer.

15. Method according to one of the preceding claims 10 to 14, **characterized in that** the hydrogen obtained in step c) is used to generate electricity at the site where the method is carried out, for example by means of a fuel cell, and **in that** the electricity generated is used to operate the plant.

16. Method according to one of the preceding claims 11 to 15, **characterized in that** the biomass is transported by a motor vehicle for hydrolysis and **in that** the hygienization provided before step a) is carried out using the waste heat of an internal combustion engine of the motor vehicle at least partially during the transport.

## Revendications

1. Installation modulaire de biogaz comprenant un certain nombre de réservoirs destinés à recevoir de la biomasse qui est transportée dans un flux massique à travers l'installation de biogaz, les réservoirs comprenant au moins deux réservoirs d'hydrolyse dans lesquels la biomasse est transformée en un hydrolysat ayant un pH inférieur à 7, les réservoirs d'hydrolyse comportant chacun une entrée et une sortie et le flux massique étant transporté de l'entrée vers la sortie et restant dans les réservoirs d'hydrolyse pendant une durée de traitement, **caractérisée en ce que** la sortie de chaque réservoir d'hydrolyse est reliée hydrauliquement à au moins un réservoir de liquide dans lequel l'hydrolysat produit peut être introduit après la transformation et y être stocké.

2. Installation modulaire de biogaz selon la revendication 1, **caractérisée en ce qu'**en amont de l'entrée, il est prévu un dispositif d'hygiénisation dans lequel la biomasse est prétraitée thermiquement avant l'entrée à une température comprise entre 70 °C et 120 °C.

3. Installation modulaire de biogaz selon la revendication 1 ou 2, **caractérisée en ce qu'**un distributeur de bactéries hydrolysantes est associé à l'entrée et/ou à chaque réservoir d'hydrolyse.

4. Installation modulaire de biogaz selon la revendication 3, **caractérisée en ce qu'**une suspension est prévue dans le distributeur pour une distribution dosée, qui contient des bactéries hydrolysantes des souches clostridium acetobutyricum, bacillus thuringiensis et/ou clostridium butyricum.

5. Installation modulaire de biogaz selon l'une des revendications précédentes,
**caractérisée en ce qu'**un distributeur de régulateurs de pH est associé à l'entrée et/ou à chaque réservoir d'hydrolyse.

6. Installation modulaire de biogaz selon la revendication 5, **caractérisée en ce que** les régulateurs de pH sont un ou plusieurs réactifs choisis parmi le groupe comprenant la soude, le bicarbonate de sodium, l'hydroxyde de sodium, l'hydroxyde de calcium, l'hydroxyde de magnésium, l'acide nitrique et l'acide chlorhydrique.

7. Installation modulaire de biogaz selon l'une des revendications précédentes, **caractérisée en ce que** les réservoirs comprennent un ou plusieurs réservoirs de fermentation qui sont disposés en aval du ou des réservoirs de liquide et qui sont reliés hydrauliquement à ceux-ci.

8. Installation modulaire de biogaz selon la revendication 7, **caractérisée en ce que** les réservoirs d'hydrolyse et les réservoirs de fermentation sont essentiellement de construction identique.

9. Installation modulaire de biogaz selon la revendication 8, **caractérisée en ce qu'**un réservoir d'hydrolyse et un réservoir de fermentation sont agencés dans un conteneur de transport standard.

10. Procédé de production de biogaz à partir de biomasse transportée dans un flux massique à travers l'installation de biogaz, **caractérisé par** les étapes suivantes :
a) collecte de biomasse sous forme de résidus végétaux, de restes alimentaires ou d'autres déchets organiques ou matières premières,
b) hydrolyse de la biomasse pendant un temps de séjour dans un certain nombre de réservoirs d'hydrolyse montés en parallèle dans le flux massique, la biomasse étant ainsi transformée en un hydrolysat ayant un pH inférieur à 7,
c) captage du gaz formé à l'étape b) et séparation de l'hydrogène produit des autres gaz, en particulier du CO₂,
d) transfert de l'hydrolysat liquide formé à l'étape c) de chacun des réservoirs d'hydrolyse dans un réservoir de liquide commun situé en aval des réservoirs d'hydrolyse,
e) acheminement de l'hydrolysat vers un ou plusieurs fermenteurs pour obtenir du méthane à partir de l'hydrolysat, en particulier avec ajout de cultures bactériennes produisant du méthane.

11. Procédé selon la revendication 10, **caractérisé en ce que,** avant l'étape a), la biomasse est prétraitée thermiquement dans un dispositif d'hygiénisation à une température comprise entre 70 °C et 120 °C.

12. Procédé selon la revendication 10 ou 11, **caractérisé en ce qu'**au début de l'étape b), des bactéries hydrolysantes sont ajoutées à la biomasse, en particulier clostridium acetobutyricum, bacillus thuringiensis et/ou clostridium butyricum.

13. Procédé selon l'une des revendications précédentes 10 à 12, **caractérisé en ce que** la biomasse est hygiénisée à la fin de l'hydrolyse et avant d'être transférée vers le réservoir de liquide.

14. Procédé selon l'une des revendications 10 à 13 précédentes, **caractérisé en ce que** l'hydrolysat est transporté à l'étape e), au moyen d'un transport par citerne, vers un dispositif générateur de méthane situé à distance de l'hydrolyseur.

15. Procédé selon l'une des revendications précédentes 10 à 14, **caractérisé en ce que** l'hydrogène obtenu à l'étape c) est utilisé sur le lieu de mise en œuvre du procédé, par exemple au moyen d'une pile à combustible pour produire de l'électricité, et **en ce que** l'électricité produite est utilisée pour faire fonctionner l'installation.

16. Procédé selon l'une des revendications 11 à 15 précédentes, **caractérisé en ce que** la biomasse est transportée vers le lieu d'hydrolyse à l'aide d'un véhicule automobile et **en ce que** l'hygiénisation prévue avant l'étape a) est réalisée au moins en partie pendant le transport en utilisant la chaleur résiduelle d'un moteur à combustion interne du véhicule à moteur.
